# EUROPEAN PATENT APPLICATION

(11) **EP 4 303 295 A1**
(43) Date of publication of application: **10.01.2024**
(21) Application number: 22775517.0
(22) Date of filing: 22.03.2022
(51) Int. Cl.: C12M 1/00, C12M 3/00

(54) **CELL CULTURE SYSTEM**

(30) Priority: 26.03.2021 JP 2021052675
(71) Applicant: TERUMO Kabushiki Kaisha, Tokyo 151-0072 (JP); Terumo BCT, Inc., Lakewood, CO 80215 (US)
(72) Inventor: IGARASHI, Masatsugu, Ashigarakami-gun, Kanagawa 259-0151 (JP); OHASHI, Hirotaka, Tokyo 163-1450 (JP)
(74) Representative: TBK
(86) International application number: PCT/JP2022/012947
(87) International publication number: WO 2022/202731

(57) **Abstract**

A cell culturing system (10) includes a reactor (12), a flow channel (16) that allows a culture medium to flow into and out of the reactor (12), a waste liquid channel (18) that discharges the culture medium from the flow channel (16), and a waste liquid container (62) capable of storing the culture medium passing through the waste liquid channel (18). The waste liquid channel (18) includes a temporary storage unit (64) capable of temporarily storing the culture medium. The waste liquid container (62) is located below the temporary storage unit (64) in a gravity direction, and
the temporary storage unit (64) is provided above the flow channel (16) in the gravity direction, temporarily stores the culture medium discharged from the flow channel (16), and allows the culture medium to flow out toward the waste liquid container (62).

## Description

### Technical Field

The present invention relates to a cell culturing system that cultures cells in a reactor by allowing a culture medium to flow into and out of the reactor.

### Background Art

In regenerative medicine, cells of a living body are collected and cultured, and the cultured cells are administered to a patient. In cell culture treatment, for example, as disclosed in JP 2017-143775 A, a cell culturing system using a cell culturing container (reactor) including a hollow fiber in a case is used. In the cell culturing system, cells are seeded in the hollow fiber of the reactor, and then a culture medium is fed into the reactor via a flow channel to culture the cells. The culture medium flowing out of the reactor during culture is discharged to a waste liquid collection container (waste liquid unit).

### Summary of Invention

In this type of cell culturing system, a waste liquid unit formed of a medical bag and the like is hung on a stand, and the waste liquid unit is arranged above the reactor in a gravity direction. As a result, the cell culturing system may apply a positive pressure to the reactor and the flow channel via the culture medium flowing into the waste liquid unit, and may suppress an excessive inflow of air (air bubbles) into the reactor.

However, in a case where a large amount of culture medium is discharged to the waste liquid unit over a long period of time, when a small-capacity medical bag is used, replacement work of the waste liquid unit is frequently required, and a workload of an operator increases. Even if the waste liquid unit is formed of a large tank and the like, the operator needs to perform an operation of removing the waste liquid unit storing a large amount of culture medium above the reactor in the gravity direction, so that a load also increases.

The present invention has been made in view of the above-described problems, and an object thereof is to provide a cell culturing system capable of appropriately applying a positive pressure to a reactor and a flow channel, and capable of reducing a workload such as replacement and removal of a waste liquid unit.

In order to achieve the above-described object, an aspect of the present invention is a cell culturing system including a reactor that cultures cells on the basis of a flow of a culture medium, a flow channel that allows the culture medium to flow into and out of the reactor, a waste liquid channel connected to the flow channel, the waste liquid channel discharging the culture medium from the flow channel, and a waste liquid container connected to the waste liquid channel, the waste liquid container being capable of storing the culture medium passing through the waste liquid channel, in which the waste liquid channel includes a temporary storage unit capable of temporarily storing the culture medium, the waste liquid container is located below the temporary storage unit in a gravity direction, and the temporary storage unit is provided above the flow channel in the gravity direction, temporarily stores the culture medium discharged from the flow channel, and allows the culture medium to flow out toward the waste liquid container.

The cell culturing system described above may appropriately apply a positive pressure to a reactor and a flow channel, and reduce a workload in replacement, removal and the like of a waste liquid unit.

### Brief Description of Drawings

Fig. 1 is a perspective view schematically illustrating an entire configuration of a cell culturing system according to an embodiment of the present invention.
Fig. 2 is a circuit diagram illustrating a flow channel and a flow path control mechanism unit between a culture medium storage unit and a reactor.
Fig. 3 is an explanatory view schematically illustrating a waste liquid channel and a waste liquid unit.
Fig. 4A is a perspective view illustrating a temporary storage unit according to a first variation. Fig. 4B is a cross-sectional view of the temporary storage unit according to the first variation.
Fig. 5 is an explanatory view schematically illustrating a waste liquid channel and a waste liquid unit according to a second variation.

### Description of Embodiments

Preferred embodiments of the present invention are hereinafter described in detail with reference to the accompanying drawings.

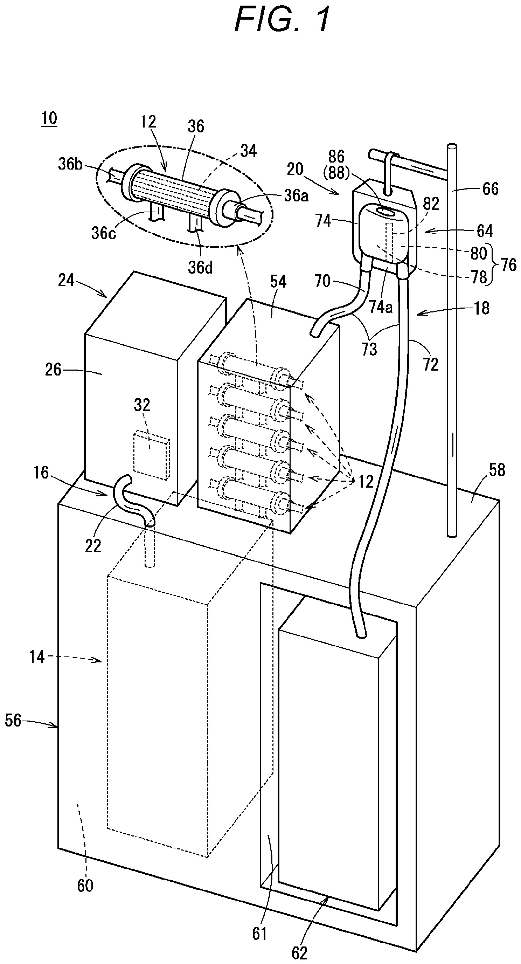

As illustrated in Fig. 1, a cell culturing system 10 according to an embodiment of the present invention is formed as a stationary device installed in a sterile room and the like, and performs culture treatment to culture cells of a living body in regenerative medicine. Therefore, the cell culturing system 10 is equipped with a reactor 12, which is a cell culturing container. The cell culturing system 10 discharges lactic acid, carbon dioxide and the like (including unused culture medium and oxygen) generated during cell culture from the reactor 12 while supplying the culture medium and oxygen to the reactor 12, thereby performing the cell culture over a long period of time.

The cells of the living body are not especially limited, and may include, for example, cells contained in the blood (T cells and the like), and stem cells (ES cells, iPS cells, mesenchymal stem cells and the like). As the culture medium, an appropriate culture medium may be selected according to the cells of the living body, and there is the culture medium prepared by adding various amino acids, vitamins, serum and the like to a balanced salt solution (BSS) as a basic solution, for example.

In addition to the reactor 12, the cell culturing system 10 includes a culture medium storage unit 14 that stores a culture medium, a flow channel 16 provided between the reactor 12 and the culture medium storage unit 14, a waste liquid channel 18 that discharges the culture medium from the flow channel 16, and a waste liquid unit 20 that stores the culture medium that flows through the waste liquid channel 18. The cell culturing system 10 according to the present embodiment is equipped with a plurality of (five in Fig. 1) reactors 12, thereby improving efficiency of the culture treatment. That is, the cell culturing system 10 is configured to obtain several times the number of cells as compared with the culture by one reactor 12 without significantly changing a culturing period by allowing the culture medium to flow through each of the plurality of reactors 12 and culturing the cells in each reactor 12.

A hard (or soft) tank capable of storing a large amount of culture medium is applied as the culture medium storage unit 14 in order to supply the culture medium to each reactor 12. For example, the tank preferably has a volume of about 5 L to 30 L, and this reduces a workload of frequently replacing the culture medium storage unit 14 during the culture treatment. A flexible medical bag and the like may be applied as the culture medium storage unit 14.

The flow channel 16 is formed of a plurality of tubes 22 (in Fig. 1, only the tube 22 connected to the culture medium storage unit 14 is illustrated). Each tube 22 is connected to the culture medium storage unit 14 and some medical bags not illustrated, and is connected to each reactor 12. As a result, the cell culturing system 10 supplies and discharges the culture medium in the culture medium storage unit 14 and the liquid (cell solution, cleaning solution, stripping solution and the like) in each medical bag to and from the reactor 12 via each tube 22.

The cell solution is a liquid containing cells seeded (to be cultured) in the reactor 12. The cleaning solution is a liquid used when priming the reactor 12 and the flow channel 16. Examples of the cleaning solution include buffer solutions such as phosphate buffered salts (PBS) and tris-buffered saline (TBS), or a physiological saline, for example. The stripping solution is a liquid that strips the cells cultured by the culture treatment. As the stripping solution, for example, trypsin, EDTA solution and the like may be used.

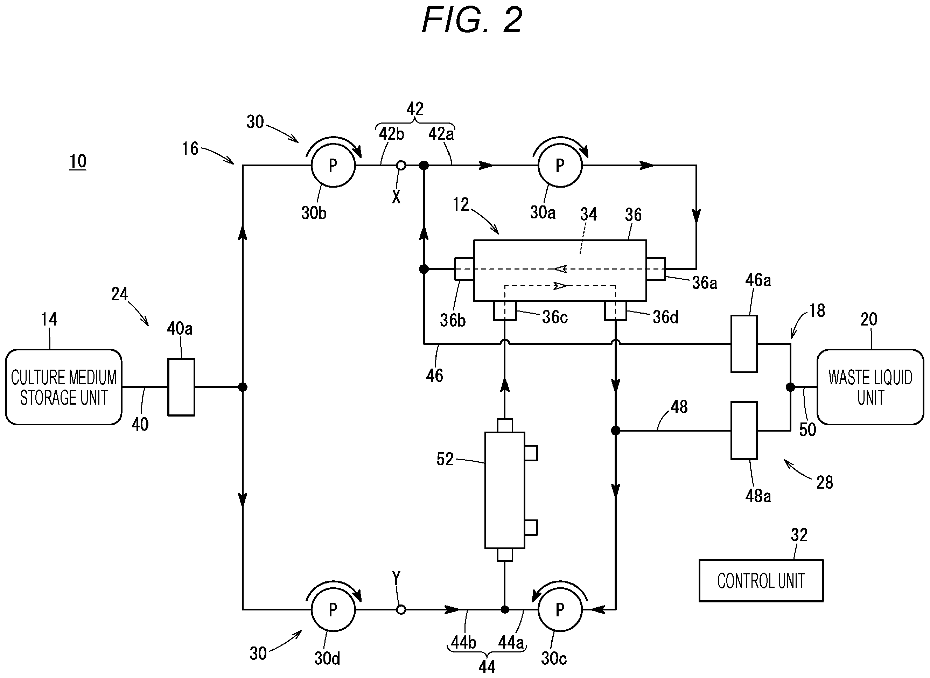

When the cell culturing system 10 is constructed, the flow channel 16 is set so as to pass through a flow path control mechanism unit 24. The flow path control mechanism unit 24 is equipped with a first casing 26 that accommodates a part of the flow channel 16. The flow path control mechanism unit 24 is equipped with a plurality of clamps 28 that opens and closes a predetermined tube 22, a plurality of pumps 30 that allows the liquid in the tube 22 to flow, and a control unit 32 that controls operations of each clamp 28 and each pump 30 in the first casing 26 (refer to Fig. 2). That is, the flow path control mechanism unit 24 allows the liquid in the flow channel 16 to flow under the operation of the pump 30 while selectively switching the tube 22 through which the liquid flows by opening and closing each clamp 28.

In addition to the plurality of tubes 22, the flow channel 16 may be equipped with a cassette (not illustrated) including a plurality of liquid flow paths, the cassette to which several tubes 22 are connected. In this case, as the cassette is set in the first casing 26, this is arranged in the clamp 28 of the flow path control mechanism unit 24, and opening/closing, switching and the like of the flow path in the cassette are performed by the clamp 28.

In order to increase a culturing area of each reactor 12 connected to the flow channel 16, for example, a structure including a hollow fiber 34 is preferably applied. Specifically, each reactor 12 is equipped with a plurality of (for example, 10,000 or more) hollow fibers 34 and a case 36 that accommodates the plurality of hollow fibers 34 in an axial direction.

Each hollow fiber 34 includes an inner cavity not illustrated penetrating in an extending direction, and cells are seeded on an inner peripheral surface forming the inner cavity. Each hollow fiber 34 includes a plurality of pores not illustrated through which the inner cavity communicates with the outside, and each pore does not permeate cells or proteins but permeates a solution or a small-molecular substance. Therefore, the culture medium, a predetermined gas component and the like are supplied to the cells on the inner peripheral surface of the hollow fiber 34 through the pores. Hereinafter, a configuration in which the liquid is allowed to mainly flow through the inner cavity of the hollow fiber 34 is also referred to as intra capillary (IC), and a configuration in which the liquid is allowed to mainly flow outside the hollow fiber 34 is also referred to as extra capillary (EC).

Examples of a material forming the hollow fiber 34 are not especially limited, and include polyolefin resins such as polypropylene and polyethylene, and polymer materials such as polysulfone, polyethersulfone, polyacrylonitrile, polytetrafluoroethylene, polystyrene, polymethylmethacrylate, cellulose acetate, cellulose triacetate, and regenerated cellulose.

The case 36 is formed into a cylindrical shape and has rigidity. The case 36 is equipped with a first IC terminal 36a, a second IC terminal 36b, a first EC terminal 36c, and a second EC terminal 36d connected to each tube 22. The first IC terminal 36a is provided at one end in the axial direction of the case 36 and communicates with the inner cavity of the hollow fiber 34. The second IC terminal 36b is provided at the other end in the axial direction of the case 36 and communicates with the inner cavity of the hollow fiber 34. The first EC terminal 36c is provided near the other end of a side surface of the case 36, and communicates with a space outside the hollow fiber 34 in the case 36. The second EC terminal 36d is provided near the one end of the side surface of the case 36, and communicates with the space outside the hollow fiber 34 in the case 36.

Hereinafter, configurations of the flow channel 16 between one reactor 12 and the culture medium storage unit 14, and the flow path control mechanism unit 24 are specifically described with reference to Fig. 2.

The flow channel 16 includes a culture medium delivery route 40 connected to the culture medium storage unit 14, and an IC route 42 (internal route) and an EC route 44 (external route) branched from the culture medium delivery route 40. The IC route 42 is a channel that supplies the liquid into the inner cavity of the hollow fiber 34, and the liquid such as the cleaning solution, cell solution, culture medium, and stripping solution flows therethrough. The EC route 44 is a channel that supplies the liquid to the outside of the hollow fiber 34 (in the case 36), and the liquid such as the cleaning solution, culture medium, and stripping solution flows therethrough.

The culture medium delivery route 40 is provided with a first clamp 40a that opens or blocks the supply of the culture medium from the culture medium storage unit 14.

The IC route 42 includes an IC circulation circuit 42a capable of circulating the liquid with the reactor 12, and an IC supply circuit 42b capable of allowing the liquid to flow from the culture medium delivery route 40 to the IC circulation circuit 42a. The IC circulation circuit 42a is provided with an IC circulation pump 30a for circulating the liquid. The IC supply circuit 42b is provided with an IC supply pump 30b for allowing the liquid to flow from the culture medium delivery route 40 to the IC circulation circuit 42a. Although not illustrated, in addition to the culture medium storage unit 14, the tube 22 connected to each medical bag storing the cleaning solution, cell solution, stripping solution and the like is connected to the IC supply circuit 42b.

The IC circulation circuit 42a is connected to the first IC terminal 36a and the second IC terminal 36b of the reactor 12. Therefore, the liquid that circulates in the IC circulation circuit 42a flows through the inner cavity of the hollow fiber 34 under the operation of the IC circulation pump 30a.

The IC waste liquid circuit 46 is connected to a downstream side from the reactor 12 on the IC circulation circuit 42a. The IC waste liquid circuit 46 forms a part of the waste liquid channel 18 and is connected to a merging route 50 of the waste liquid channel 18. The IC waste liquid circuit 46 is provided with a second clamp 46a that opens or blocks discharge of the liquid from the IC circulation circuit 42a.

In contrast, the EC route 44 includes an EC circulation circuit 44a capable of circulating the liquid with the reactor 12, and an EC supply circuit 44b capable of allowing the liquid to flow from the culture medium delivery route 40 to the EC circulation circuit 44a. The EC circulation circuit 44a is provided with an EC circulation pump 30c for circulating the liquid. The EC supply circuit 44b is provided with an EC supply pump 30d for allowing the liquid to flow from the culture medium delivery route 40 to the EC circulation circuit 44a. Although not illustrated, in addition to the culture medium storage unit 14, the tube 22 connected to each medical bag storing the cleaning solution, stripping solution and the like is connected to the EC supply circuit 44b.

The EC circulation circuit 44a is connected to the first EC terminal 36c and the second EC terminal 36d of the reactor 12. Therefore, the liquid that circulates in the EC circulation circuit 44a flows through the case 36 under the operation of the EC circulation pump 30c. A gas exchanger 52 is provided on an upstream side from the reactor 12 on the EC circulation circuit 44a. The gas exchanger 52 has a function of discharging carbon dioxide mixed in the culture medium and mixing a predetermined gas component (nitrogen N₂: 75%, oxygen O₂: 20%, carbon dioxide CO₂: 5%) with the culture medium. A structure of the gas exchanger 52 is not especially limited, and a structure in which a plurality of hollow fibers is provided in the case may be applied as is the case with the reactor 12.

An EC waste liquid circuit 48 is connected to a downstream side from the reactor 12 on the EC circulation circuit 44a. The EC waste liquid circuit 48 forms a part of the waste liquid channel 18 and is connected to the merging route 50 of the waste liquid channel 18. The EC waste liquid circuit 48 is provided with a third clamp 48a that opens or blocks discharge of the liquid from the EC circulation circuit 44a.

As described above, in a case where a plurality of (five) reactors 12 is provided, the cell culturing system 10 may be equipped with a plurality of IC circulation circuits 42a and a plurality of EC circulation circuits 44a corresponding to the respective reactors 12. That is, another IC circulation circuit and another EC circulation circuit not illustrated that circulate the liquid in another reactor 12 are connected in parallel to a branch point X between the IC supply pump 30b and the IC circulation circuit 42a and a branch point Y between the EC supply pump 30d and the EC circulation circuit 44a, respectively.

With reference to Fig. 1 again, the cell culturing system 10 includes a second casing 54 that accommodates each reactor 12 at a position adjacent to the first casing 26 that forms the flow path control mechanism unit 24. The second casing 54 has a function of keeping temperature of an accommodation chamber of each reactor 12 at 37°C. That is, the cell culturing system 10 may easily form an environment suitable for the cell culture of each reactor 12 by using the second casing 54 different from the first casing 26 of the flow path control mechanism unit 24. The cell culturing system 10 is not limited to the configuration in which each reactor 12 and the flow channel 16 are accommodated in a plurality of casings, and may be configured to accommodate them in one casing.

The second casing 54 may be formed to include a part (clamp 28, pump 30 and the like) of the flow path control mechanism unit 24 therein. For example, the second clamp 46a on the IC waste liquid circuit 46 and the third clamp 48a on the EC waste liquid circuit 48 are provided in the second casing 54. The second casing 54 is preferably configured to rotatably fix each reactor 12 in a gravity direction or a horizontal direction, or around the axis of the case 36. As a result, air in each reactor 12 is easily discharged from the case 36.

Furthermore, the cell culturing system 10 is equipped with an installation base 56 on which the first casing 26 and the second casing 54 are installed. The installation base 56 includes a top plate 58 on which the first casing 26 and the second casing 54 are placed, and the top plate 58 is supported at a predetermined height (about 50 cm to 150 cm) by a side wall and the like of the installation base 56. The culture medium storage unit 14 is accommodated in a culture medium accommodation box 60 of the installation base 56 provided below the top plate 58.

The waste liquid channel 18 of the cell culturing system 10 is connected to the flow channel 16 described above and is connected to the waste liquid unit 20, thereby discharging the liquid such as the culture medium, the cleaning solution and the like from the flow channel 16 to the waste liquid unit 20. The waste liquid channel 18 includes the IC waste liquid circuit 46 of the IC route 42, the EC waste liquid circuit 48 of the EC route 44, and the merging route 50 (refer to Fig. 2). The merging route 50 of the waste liquid channel 18 is provided so as to be extended from the interior of the second casing 54 (or the first casing 26) to the outside.

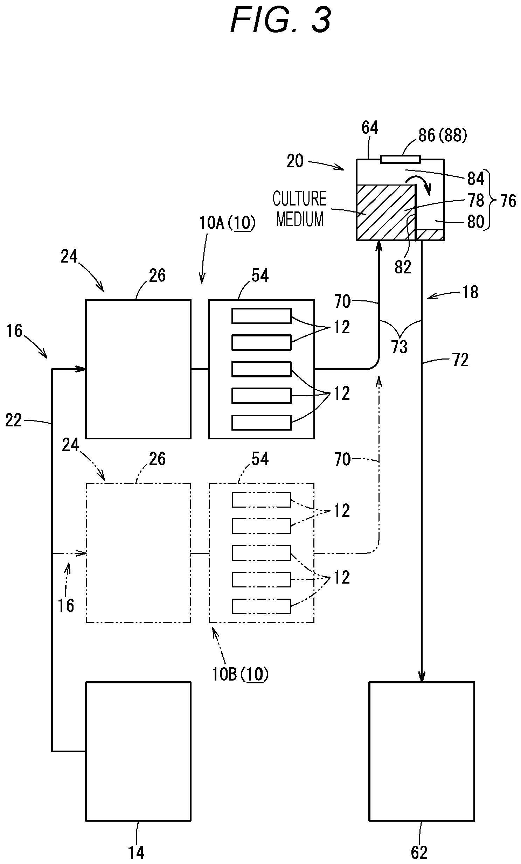

As illustrated in Figs. 1 and 3, the waste liquid unit 20 includes a waste liquid container 62 that is connected to the most downstream side of the waste liquid channel 18 and capable of storing the culture medium passing through the waste liquid channel 18. The waste liquid channel 18 includes a temporary storage unit 64 capable of temporarily storing the culture medium on an upstream side from the waste liquid container 62.

In the waste liquid container 62, a hard (or soft) tank having a large volume is applied for storing the culture medium used in each reactor 12. For example, the tank preferably has the volume of about 5 L to 30 L. As a result, a workload of frequently replacing the waste liquid container 62 is reduced. Alternatively, a flexible medical bag and the like may be applied as the waste liquid container 62.

The waste liquid container 62 is accommodated in a waste liquid accommodation box 61 of the installation base 56 provided below the top plate 58. That is, the waste liquid container 62 is provided below the temporary storage unit 64 in the gravity direction. The waste liquid container 62 according to the present embodiment is located below the first casing 26 and the second casing 54 accommodating each reactor 12 in the gravity direction. Although Fig. 1 illustrates a state in which the waste liquid container 62 is exposed from the waste liquid accommodation box 61, the waste liquid accommodation box 61 may have a configuration of hermetically sealing the waste liquid container 62.

In contrast, the temporary storage unit 64 temporarily stores the liquid discharged from the flow channel 16 and then allows the same to flow out toward the waste liquid container 62. Therefore, a volume of the temporary storage unit 64 is sufficiently smaller than a volume of the waste liquid container 62. The temporary storage unit 64 is, for example, hung on a stand 66 fixed to the installation base 56, and is located above the first casing 26 and the second casing 54 accommodating the plurality of reactors 12 in the gravity direction. In other words, the temporary storage unit 64 is arranged above the reactor 12 and the flow channel 16 in the gravity direction. A height of the temporary storage unit 64 is not especially limited, and this may be set in a range of 150 cm to 180 cm, for example, and is set to have a predetermined difference (range of 10 cm to 80 cm) with respect to the heights of the reactor 12 and the flow channel 16.

The waste liquid channel 18 (merging route 50) includes an upstream line 70 provided between the flow channel 16 and the temporary storage unit 64 via the IC waste liquid circuit 46 and the EC waste liquid circuit 48, and a downstream line 72 provided between the temporary storage unit 64 and the waste liquid container 62. The upstream line 70 and the downstream line 72 are formed of a tube 73 including a flow path inside. The upstream line 70 is extended upward in the gravity direction from the second casing 54 and is connected to a lower portion of the temporary storage unit 64. The downstream line 72 is extended downward in the gravity direction from the temporary storage unit 64 and is connected to an upper portion of the waste liquid container 62.

A flexible medical bag is applied as the temporary storage unit 64. The temporary storage unit 64 includes a sealed portion 74 obtained by sealing outer peripheries of two sheets forming the medical bag, and includes a storage space 76 inside the sealed portion 74 and between the two sheets. The upstream line 70 and the downstream line 72 are coupled to the sealed portion 74 on a lower side of the temporary storage unit 64 (hereinafter, referred to as a lower sealed portion 74a). The temporary storage unit 64 may be formed of a hard container.

A partition wall 82 that separates a lower side of the storage space 76 into a first storage unit 78 and a second storage unit 80 is provided inside the temporary storage unit 64. The partition wall 82 is continued to the lower sealed portion 74a and is extended upward in the gravity direction from the lower sealed portion 74a. The partition wall 82 is formed by, for example, sealing the two sheets forming the medical bag. Alternatively, the partition wall 82 may be formed by welding an edge of a plate member and each sheet in a state in which the plate member having a predetermined thickness in a direction orthogonal to two sheet surfaces is interposed between the two sheets.

In the temporary storage unit 64, a communication unit 84 (a part of the storage space 76) through which the first storage unit 78 communicates with the second storage unit 80 is provided above the partition wall 82 in the gravity direction. That is, the storage space 76 is formed of the communication unit 84 on the upper side in the gravity direction, and the first storage unit 78 and the second storage unit 80 adjacent to each other in a lateral direction (direction orthogonal to the gravity direction) of the partition wall 82 below the communication unit 84 in the gravity direction. A flow path of the upstream line 70 fixed to the lower sealed portion 74a communicates with the first storage unit 78. A flow path of the downstream line 72 fixed to the lower sealed portion 74a communicates with the second storage unit 80.

Therefore, the liquid that flows into the temporary storage unit 64 from the upstream line 70 is first stored in the first storage unit 78, and when the liquid fills the first storage unit 78, the liquid goes over the partition wall 82 and flows into the second storage unit 80. The liquid that flows into the second storage unit 80 flows out to the downstream line 72 (outside the temporary storage unit 64).

The first storage unit 78 is configured to apply an appropriate pressure (positive pressure) to the reactor 12 and the flow channel 16 via the stored culture medium. For example, a volume of the first storage unit 78 is preferably set in a range of about 0.5 times to three times a volume of the second storage unit 80. An actual volume of the first storage unit 78 may be set to, for example, a range of 50 cc to 300 cc.

The temporary storage unit 64 includes an atmosphere open unit 86 that applies an atmospheric pressure to the liquid that flows into the first storage unit 78. In the present embodiment, the atmosphere open unit 86 includes a vent mechanism 88 that permeates gas and blocks permeation of a liquid. As a result, the vent mechanism 88 may apply the atmospheric pressure to the liquid in the first storage unit 78 without leaking the liquid that flows into the storage space 76 to the outside. The atmosphere open unit 86 is not limited to the vent mechanism 88, and may be formed of an opening that simply opens an upper side in the gravity direction of the temporary storage unit 64.

As illustrated in Fig. 3, the waste liquid unit 20 may be connected to a plurality of cell culturing systems 10. For example, by branching the upstream line 70 of the waste liquid channel 18, the flow channel 16 of a first cell culturing system 10A (solid line in Fig. 3) and the flow channel 16 of a second cell culturing system 10B (two-dot chain line in Fig. 3) are connected to the temporary storage unit 64. Therefore, the waste liquid unit 20 temporarily stores both the liquid that flows out of the flow channel 16 of the first cell culturing system 10A and the liquid that flows out of the flow channel 16 of the second cell culturing system 10B in one temporary storage unit 64. Then, the liquid stored in the temporary storage unit 64 is discharged to one or more waste liquid containers 62 via the downstream line 72.

The cell culturing system 10 according to the present embodiment is basically formed as described above, and an operation thereof is hereinafter described.

As illustrated in Fig. 1, in the cell culturing system 10, before the culture treatment is performed, an operator sets a plurality of reactors 12 in the second casing 54 and sets the flow channel 16 in the flow path control mechanism unit 24. The operator accommodates the culture medium storage unit 14 in the culture medium accommodation box 60 of the installation base 56, and installs the waste liquid container 62 in the waste liquid accommodation box 61 of the installation base 56, whereas the temporary storage unit 64 is hung on the stand 66. As a result, the flow channel 16 illustrated in Fig. 2 is constructed between the culture medium storage unit 14 and each reactor 12, and the temporary storage unit 64 is arranged above the flow channel 16 in the gravity direction.

After the above-described setting, the cell culturing system 10 sequentially performs a priming step, a culture medium replacement step, a seeding step, a culturing step, a stripping step, and a collection step in the culture treatment. At the priming step, the cleaning solution stored in a medical bag not illustrated is supplied to each reactor 12 via the flow channel 16, and air is removed from the reactor 12 and the flow channel 16. At the culture medium replacement step, the culture medium is supplied from the culture medium storage unit 14 to each reactor 12 via the primed flow channel 16, and the inside and outside of the hollow fiber 34 are filled with the culture medium. At the seeding step, the cell solution stored in a medical bag not illustrated is supplied into the hollow fiber 34 of each reactor 12 via the IC route 42, and the cells are seeded on the inner peripheral surface of the hollow fiber 34.

Then, as illustrated in Fig. 2, at the culturing step, the cell culturing system 10 supplies the culture medium from the culture medium storage unit 14 into the hollow fiber 34 via both the IC route 42 and the EC route 44, and cultures the cells in the hollow fiber 34. At that time, carbon dioxide is discharged from the culture medium and oxygen is supplied to the culture medium by the gas exchanger 52. The culturing step is performed for a longer period of time (for example, several days) than other steps, so that the cells gradually propagates on the inner peripheral surface of the hollow fiber 34. The cell culturing system 10 may be configured to supply the culture medium to the reactor 12 via the EC route 44 without passing through the IC supply circuit 42b. The culture medium that flows through the EC route 44 to flow into the reactor 12 oozes from the outside to the inside of the hollow fiber 34 to be supplied to the cells.

At the culturing step, the culture medium that circulates in the IC circulation circuit 42a flows into the IC waste liquid circuit 46 while the second clamp 46a is opened. The culture medium that circulates in the EC circulation circuit 44a flows into the EC waste liquid circuit 48 while the third clamp 48a is opened. As a result, the culture medium flows through the waste liquid channel 18. The culture medium in the IC waste liquid circuit 46 and the EC waste liquid circuit 48 moves outside the second casing 54 by flowing into the upstream line 70 of the merging route 50. This culture medium flows upward in the gravity direction via the upstream line 70 and flows into the first storage unit 78 of the temporary storage unit 64.

As illustrated in Fig. 3, the temporary storage unit 64 continuously stores the culture medium in the first storage unit 78 until the culture medium exceeds the partition wall 82. When the culture medium in the first storage unit 78 exceeds the partition wall 82, the culture medium flows over the partition wall 82 (via the communication unit 84) to flow into the second storage unit 80. The culture medium moved to the second storage unit 80 flows to the downstream line 72 fixed to a lower portion of the second storage unit 80. That is, when an inflow amount of the culture medium exceeds a certain amount, the temporary storage unit 64 automatically discharges the culture medium to the downstream line 72 below the same.

The culture medium discharged to the downstream line 72 flows downward in the gravity direction and flows into the waste liquid container 62 installed on the lower side of the installation base 56. The waste liquid container 62 has a volume capable of sufficiently storing the culture medium, and may significantly reduce the number of times of replacement of the waste liquid container 62 at the culturing step.

Then, the temporary storage unit 64 arranged above the flow channel 16 and the reactor 12 in the gravity direction may apply the positive pressure to the flow channel 16 via the culture medium in the first storage unit 78 and the upstream line 70. Therefore, in the EC circulation circuit 44a, the positive pressure of the culture medium is applied, so that it is possible to suppress an excessive inflow of air in the gas exchanger 52 and to stably mix the air and the culture medium. As a result, in the cell culturing system 10, the inflow of air bubbles into the flow channel 16 is significantly suppressed.

The atmosphere open unit 86 provided in the temporary storage unit 64 may apply the atmospheric pressure to the culture medium in the first storage unit 78 and the upstream line 70, thereby applying the positive pressure to the flow channel 16 even in a case where the amount of the culture medium is small. Especially, since the vent mechanism 88 is employed as the atmosphere open unit 86, it is possible to avoid leakage of the culture medium from the temporary storage unit 64.

At the stripping step after the culturing step, the cell culturing system 10 guides the stripping solution stored in a medical bag not illustrated into the hollow fiber 34 of the reactor 12 via the IC route 42, and strips the cultured (propagated) cells. At the collection step after the stripping step, the cell culturing system 10 supplies the culture medium to the IC route 42 to allow the cells stripped at the stripping step to flow out of the reactor 12 and move to a collection bag not illustrated.

Through the above-described steps, the cell culturing system 10 may satisfactorily store the cells cultured in the reactor 12 in the collection bag. Especially, the cell culturing system 10 may stably apply the positive pressure to the flow channel 16, and may reduce a workload of the operator by reducing the number of times of replacement, removal and the like of the waste liquid container 62 even when a large amount of culture medium is used.

The present invention is not limited to the above-described embodiment, and various modifications may be made in accordance with the gist of the invention. For example, the cell culturing system 10 may be configured to perform the culture treatment by one reactor 12 without using a plurality of reactors 12. In a case of increasing the number of cultured cells in the culture treatment, a large-sized reactor 12 may be applied.

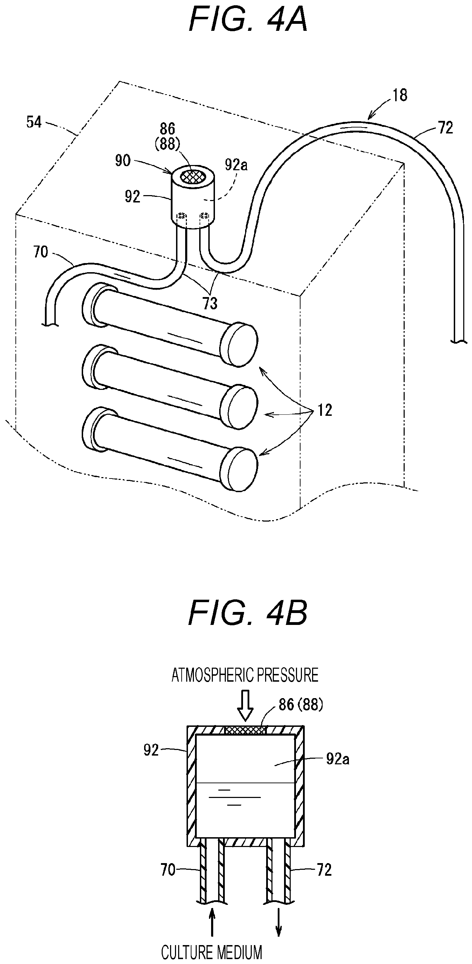

As in a first variation illustrated in Figs. 4A and 4B, the temporary storage unit 90 may be provided on an upper side in the second casing 54 that accommodates a plurality of reactors 12 (or the first casing 26: refer to Fig. 1). In this case also, the temporary storage unit 90 is arranged above the reactor 12 and the flow channel 16 in the gravity direction. For example, the temporary storage unit 90 is formed of a small and hard container 92 in such a manner that a volume does not change in the second casing 54 (other mechanisms are not pressed by the flexible bag).

The partition wall 82 (refer to Fig. 1) is not provided inside the container 92, and a storage space 92a enclosed by an inner surface of the container 92 is formed. Each of the upstream line 70 and the downstream line 72 of the waste liquid channel 18 is connected to a lower end of the container 92 of the temporary storage unit 90. The upstream line 70 is extended upward in the gravity direction from a connection site of the flow channel 16 (refer to Fig. 2). In contrast, the downstream line 72 temporarily directed upward in the gravity direction from a lower portion of the container 92 in the second casing 54, thereby being exposed outside the second casing 54. Then, outside the second casing 54, the downstream line 72 is extended downward in the gravity direction and is connected to the waste liquid container 62 (refer to Fig. 1) installed below the second casing 54 in the gravity direction.

Furthermore, the atmosphere open unit 86 that applies the atmospheric pressure to the culture medium that flows into the storage space 92a is provided on an upper end of the container 92 of the temporary storage unit 90. The atmosphere open unit 86 includes the vent mechanism 88 that permeates gas and blocks permeation of a liquid. The atmosphere open unit 86 may have a configuration in which the upper end of the container 92 is simply opened.

The temporary storage unit 90 formed as described above may also obtain an effect similar to that of the temporary storage unit 64 described above. That is, the culture medium that flows into the temporary storage unit 90 from the flow channel 16 via the upstream line 70 is temporarily stored in the storage space 92a of the container 92. When the atmospheric pressure is applied to the culture medium in the container 92 from the atmosphere open unit 86, the positive pressure may be applied to the flow channel 16 via the culture medium of the upstream line 70. Especially, the temporary storage unit 90 provided in the second casing 54 may avoid disadvantages such as careless dropping of the temporary storage unit 90 by an operator or the like.

The culture medium in the storage space 92a is guided to the downstream line 72 by a siphon effect. The culture medium is temporarily directed upward in the gravity direction in the downstream line 72, then guided downward in the gravity direction, and stored in the waste liquid container 62. As a result, the culture medium is temporarily stored in the temporary storage unit 90 and then smoothly discharged to the waste liquid container 62, and does not accumulate in a large amount in the container 92.

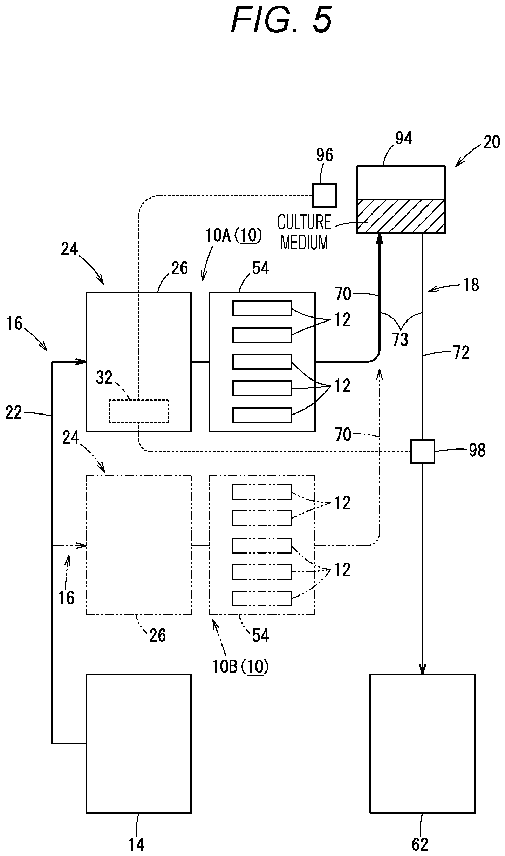

Furthermore, as in a second variation illustrated in Fig. 5, the cell culturing system 10 may apply a temporary storage unit 94 without the partition wall 82, and include a sensor 96 that detects a weight or a liquid level of the temporary storage unit 94, and a valve 98 that opens and closes the downstream line 72. The sensor 96 and the valve 98 are connected to the control unit 32 of the cell culturing system 10 so as to be able to communicate by wire or wirelessly. The control unit 32 closes the valve 98 in a normal state, monitors the amount of liquid flowing into the temporary storage unit 94 on the basis of detection information of the sensor 96, and opens the valve 98 in a case where the amount of liquid exceeds a predetermined threshold. Even in this case, the cell culturing system 10 may stably apply the positive pressure to the flow channel 16.

Technical ideas and effects that may be grasped from the above-described embodiment are described as follows.

An aspect of the present invention is a cell culturing system 10 including a reactor 12 that cultures cells on the basis of a flow of a culture medium, a flow channel 16 that allows the culture medium to flow into and out of the reactor 12, a waste liquid channel 18 connected to the flow channel 16, the waste liquid channel 18 discharging the culture medium from the flow channel 16, and a waste liquid container 62 connected to the waste liquid channel 18, the waste liquid container 62 being capable of storing the culture medium passing through the waste liquid channel 18, in which the waste liquid channel 18 includes a temporary storage unit 64, 90, 94 capable of temporarily storing the culture medium, the waste liquid container 62 is located below the temporary storage unit 64, 90, 94 in a gravity direction, and the temporary storage unit 64, 90, 94 is provided above the flow channel 16 in the gravity direction, temporarily stores the culture medium discharged from the flow channel 16, and allows the culture medium to flow out toward the waste liquid container 62.

According to the description above, in the cell culturing system 10, the temporary storage unit 64, 90, 94 is located above the flow channel 16 in the gravity direction, so that the positive pressure may be appropriately applied to the reactor 12 and the flow channel 16 via the culture medium of the temporary storage unit 64, 90, 94. As a result, the cell culturing system 10 may suppress the excessive inflow of air into the reactor 12 and the flow channel 16. Since the waste liquid container 62 is located below the temporary storage unit 64, 90, 94 in the gravity direction, the cell culturing system 10 may reduce a workload in replacement, removal and the like of the waste liquid container 62.

The waste liquid container 62 is provided below an installation site of the reactor 12 in the gravity direction. This makes it easier for the operator of the cell culturing system 10 to perform operations such as replacement and removal of the waste liquid container 62.

A first storage unit 78, a second storage unit 80, a partition wall 82 that partitions the first storage unit 78 and the second storage unit 80, and a communication unit 84 through which the first storage unit 78 communicates with the second storage unit 80 above the partition wall 82 in the gravity direction are provided inside the temporary storage unit 64, 94, the waste liquid channel 18 includes an upstream line 70 through which the flow channel 16 communicates with the first storage unit 78, and a downstream line 72 through which the second storage unit 80 communicates with the waste liquid container 62, and the downstream line 72 is extended downward in the gravity direction from the second storage unit 80. As a result, the culture medium stored in the first storage unit 78 may apply an appropriate positive pressure to the reactor 12 and the flow channel 16.

The first storage unit 78 and the second storage unit 80 are located at positions adjacent to each other in a direction orthogonal to the gravity direction, and a volume of the first storage unit 78 is larger than a volume of the second storage unit 80. As a result, the temporary storage unit 64 may apply a large positive pressure from the first storage unit 78 to the reactor 12 and the flow channel 16.

The temporary storage unit 90 includes an atmosphere open unit 86 that applies an atmospheric pressure to the culture medium that flows into the temporary storage unit 90, and the atmosphere open unit 86 includes a vent mechanism 88 that permeates gas and blocks permeation of a liquid. As a result, even in the configuration in which the cell culturing system 10 includes the atmosphere open unit 86, leakage of the culture medium from the temporary storage unit 90 is suppressed.

The waste liquid channel 18 includes an upstream line 70 through which the flow channel 16 communicates with the temporary storage unit 90, and a downstream line 72 through which the temporary storage unit 90 communicates with the waste liquid container 62, and the downstream line 72 is temporarily directed upward in the gravity direction from a lower portion of the temporary storage unit 90, then directed downward in the gravity direction. Even in this case, the cell culturing system 10 may discharge the culture medium from the temporary storage unit 90 to the waste liquid container 62 while applying the positive pressure to the reactor 12 and the flow channel 16 by the culture medium stored in the temporary storage unit 90.

The temporary storage unit 64, 94 is arranged outside a casing (second casing 54) that accommodates the reactor 12. As a result, the operator may easily set the temporary storage unit 64, 94 when preparing the cell culturing system 10. The operator may visually check the culture medium in the temporary storage unit 64, 94 located outside and recognize a waste liquid state of the culture medium.

The temporary storage unit 90 is arranged inside a casing (second casing 54) that accommodates the reactor 12. As a result, the cell culturing system 10 may avoid disadvantages such as careless dropping of the temporary storage unit 90 by the operator or the like.

The flow channel 16 includes a circulation circuit (EC circulation circuit 44a) that circulates the culture medium with the reactor 12, and a supply circuit (EC supply circuit 44b) that supplies the culture medium to the circulation circuit, and the circulation circuit includes a gas exchanger 52 that mixes gas with the culture medium on an upstream side from the reactor 12 in a flow direction of the culture medium, and the waste liquid channel 18 is connected to a downstream side from the reactor 12 in the flow direction of the culture medium. As a result, the cell culturing system 10 may apply an appropriate positive pressure to the circulation circuit including the gas exchanger 52 via the culture medium in the temporary storage unit 64, 90, 94 and the waste liquid channel 18, and may suppress an excessive inflow of gas in the gas exchanger 52.

A plurality of reactors 12 is provided, and the culture medium that flows through the plurality of reactors 12 is collectively discharged to the waste liquid channel 18 and the waste liquid container 62. The cell culturing system 10 may efficiently culture the cells by the plurality of reactors 12. The cell culturing system 10 may stably waste the culture medium by the waste liquid container 62 and the temporary storage unit 64, 90, 94 by using the plurality of reactors 12 even if a large amount of the culture medium flows.

## Claims

1. A cell culturing system comprising:
a reactor that cultures cells on the basis of a flow of a culture medium;
a flow channel that allows the culture medium to flow into and out of the reactor;
a waste liquid channel connected to the flow channel, the waste liquid channel discharging the culture medium from the flow channel; and
a waste liquid container connected to the waste liquid channel, the waste liquid container being capable of storing the culture medium passing through the waste liquid channel, wherein
the waste liquid channel includes a temporary storage unit capable of temporarily storing the culture medium,
the waste liquid container is located below the temporary storage unit in a gravity direction, and
the temporary storage unit is provided above the flow channel in the gravity direction, temporarily stores the culture medium discharged from the flow channel, and allows the culture medium to flow out toward the waste liquid container.

2. The cell culturing system according to claim 1, wherein
the waste liquid container is provided below an installation site of the reactor in the gravity direction.

3. The cell culturing system according to claim 1 or 2, wherein
a first storage unit, a second storage unit, a partition wall that partitions the first storage unit and the second storage unit, and a communication unit through which the first storage unit communicates with the second storage unit above the partition wall in the gravity direction are provided inside the temporary storage unit,
the waste liquid channel includes an upstream line through which the flow channel communicates with the first storage unit, and a downstream line through which the second storage unit communicates with the waste liquid container, and
the downstream line is extended downward in the gravity direction from the second storage unit.

4. The cell culturing system according to claim 3, wherein
the first storage unit and the second storage unit are at positions adjacent to each other in a direction orthogonal to the gravity direction, and a volume of the first storage unit is larger than a volume of the second storage unit.

5. The cell culturing system according to claim 1 or 2, wherein
the temporary storage unit includes an atmosphere open unit that applies an atmospheric pressure to the culture medium that flows into the temporary storage unit, and
the atmosphere open unit includes a vent mechanism that permeates gas and blocks permeation of a liquid.

6. The cell culturing system according to claim 5, wherein
the waste liquid channel includes an upstream line through which the flow channel communicates with the temporary storage unit, and a downstream line through which the temporary storage unit communicates with the waste liquid container, and
the downstream line is temporarily directed upward in the gravity direction from a lower portion of the temporary storage unit, then directed downward in the gravity direction.

7. The cell culturing system according to any one of claims 1 to 6, wherein
the temporary storage unit is arranged outside a casing that accommodates the reactor.

8. The cell culturing system according to any one of claims 1 to 6, wherein
the temporary storage unit is arranged inside a casing that accommodates the reactor.

9. The cell culturing system according to any one of claims 1 to 8, wherein
the flow channel includes:
a circulation circuit that circulates the culture medium with the reactor; and
a supply circuit that supplies the culture medium to the circulation circuit, and
the circulation circuit includes a gas exchanger that mixes gas with the culture medium on an upstream side from the reactor in a flow direction of the culture medium, and the waste liquid channel is connected to a downstream side from the reactor in the flow direction of the culture medium.

10. The cell culturing system according to any one of claims 1 to 9, wherein
a plurality of reactors is provided, and
the culture medium that flows through the plurality of reactors is collectively discharged to the waste liquid channel and the waste liquid container.
